# EUROPÄISCHE PATENTSCHRIFT

(11) **EP 0 691 324 B1**
(45) Veröffentlichungstag und Bekanntmachung des Hinweises auf die Patenterteilung: **16.09.1998**
(21) Anmeldenummer: 95109577.7
(22) Anmeldetag: 21.06.1995
(51) Int. Cl.: C07C 67/08, C07C 69/60

(54) **Verfahren zur Herstellung von Maleinsäuredialkylestern**
Process for the preparation of dialkyl esters of maleic acid
Procédé de préparation d'esters dialkyliques d'acide maléique

(30) Priorität: 04.07.1994 DE 4423355
(43) Veröffentlichungstag der Anmeldung: 10.01.1996
(73) Patentinhaber: BAYER AG, 51368 Leverkusen (DE)
(72) Erfinder: Müller, Nikolaus, Dr., D-40789 Monheim (DE); Gröschl, Andreas, Dr., D-51375 Leverkusen (DE); Janisch, Ingo, Dr., Groton MA 01450 (US)

(56) Entgegenhaltungen:
- EP-A- 0 476 370
- DE-A- 4 019 170

## Beschreibung

Die vorliegende Erfindung betrifft ein Verfahren zur Herstellung von Maleinsäuredialkylestern, insbesondere Maleinsäurediethylester, durch Veresterung von Maleinsäureanhydrid mit Alkoholen bei der Siedetemperatur der Alkoholkomponente, wobei das entstehende Dampfgemisch aus Alkohol/Wasser an einer Membran getrennt wird und die entwässerte Alkoholkomponente in das Reaktionsgemisch zurückgeführt wird.

Es ist bereits bekannt, Maleinsäuredialkylester durch Veresterung von Maleinsäureanhydrid mit überschüssigem Alkohol herzustellen (s. DE-A 3 114 320, DD-A 229 117). Bei dieser Reaktion handelt es sich wie bei allen Veresterungen um eine Gleichgewichtsreaktion, die nur dann zu befriedigenden Ausbeuten führt, wenn Maßnahmen zur Verschiebung des Gleichgewichts ergriffen werden. Hierzu gehört die Wasserentfernung während der Reaktion durch azeotrope Destillation mit überschüssigem Alkohol oder einem inerten Schleppmittel wie Toluol. Der Nachteil dieses Verfahrens ist der Verbrauch großer Mengen Alkohol, da niedere Alkohole mit Wasser völlig mischbar sind und deshalb nicht durch Phasentrennung des Destillats zurückgeführt werden können. Für eine Rückgewinnung des Alkohols müssen daher spezielle Entwässerungsmethoden angewandt werden.

Bei Verwendung eines inerten Lösungsmittels als Schleppmittel treten in dem System Wasser/Alkohol/Schleppmittel ebenfalls Trennprobleme auf, so daß auch hier das Destillat in der Regel nicht direkt in das Reaktionsgemisch zurückgeführt werden kann. Beide Methoden der Wasserauskreisung führen zu verlängerten Reaktionszeiten, was sich bei Maleinsäuredialkylestern in einer verstärkten Isomerisierung zu Fumarsäureestern negativ bemerkbar macht (DE-A 3 114 320 ).

Zur Erreichung höherer Reaktionsgeschwindigkeiten kann bei Temperaturen oberhalb des Siedepunktes der am niedrigsten siedenden Komponente unter Druck gearbeitet werden. Solche höheren Temperaturen führen in der Regel ebenfalls zu Isomerisierung und zur Bildung weiterer Nebenkomponenten, wie Alkoxybernsteinsäureestern, die destillativ nur unter großem Trennaufwand zu entfernen sind (DE-A 3 114 320).

Bei der Herstellung von Maleinsäuredialkylestern ist ferner von Bedeutung, den als Zwischenstufe gebildeten Maleinsäurehalbester möglichst vollständig zu entfernen, da eine destillative Trennung vom Diester zur thermischen Rückreaktion des Halbesters unter Bildung von Maleinsäureanhydrid und Alkohol führt, was wiederum zu völlig unübersichtlichen Stoffgemischen führt. Die zur Umgehung dieses Nachteils beschriebenen Kontiprozesse erfordern einen hohen technischen Aufwand mit komplexen Rückführungsvorgängen (EP-A 255 399, EP-A 255 401).

Es ist weiterhin bekannt bei chemischen Gleichgewichtsreaktionen, bei denen Wasser gebildet wird, dieses mit Hilfe von semipermeablen Membranen abzutrennen und die Reaktion so zu vervollständigen. Solche Membranverfahren zur Fraktionierung von Gemischen mit organischen Komponenten sind in der Literatur ausführlich beschrieben (Rautenbach, Chem. Ing. Techn. 61 (1989) S. 539 - 544).

Pervaporation und Dampfpermeation wurden ebenfalls schon ausführlich beschrieben (DE 3 610 011, DE-A-4 019 170, EP-A 273 267 und EP-A-294 827).

Generell wird bei diesen Verfahren das zu trennende Stoffgemisch (Feed) an einer Membran entlanggeführt, die für die einzelnen Verbindungen des zulaufenden Stoffgemisches unterschiedliche Permeabilitäten aufweist. Triebkraft für den Stofftransport durch die Membran ist dabei eine transmembrane Differenz des elektrochemischen Potentials der einzelnen Stoffe des Zulaufgemisches. Im Falle der Pervaporation und Dampfpermeation wird diese Potentialdifferenz durch ein an der dem Feed abgewandten Seite der Membran (Permeatseite) angelegtes Vakuum oder durch Spülen der Permeatseite mit Inertgas aufgeprägt, was ein Ausschleusen der bevorzugt permeierenden Komponenten aus dem Feed zur Folge hat. Der entreicherte Feed wird als Retentat und die auf die Permeatseite übergehenden Stoffmengen als Permeat bezeichnet.

Das Trennverhalten von Membranen ist stark temperaturabhängig, wird aber durch die thermische Beständigkeit der eingesetzten Membran begrenzt. So wird beispielsweise die Temperaturbelastbarkeit der Membran Pervap 1 000 der Firma GFT vom Typ Polyvinylalkohol/Polyacrylnitril mit 100°C angegeben.

Die in DE-A-3 610 011 und EP-A-240 803 beschriebenen Verfahren zeichnen sich dadurch aus, daß der Feed in flüssiger Form knapp unterhalb des Siedepunktes bei gegebenem Systemdruck zugeführt wird (Pervaporation). Der Systemdruck muß dabei so gewählt werden, daß die Temperatur des zu trennenden Stoffgemisches an der Membran möglichst der optimalen Temperatur hinsichtlich Permeatfluß und Selektivität entspricht, keinesfalls aber oberhalb der maximal zulässigen Betriebstemperatur der Membran liegen darf (Membranbeständigkeit).

Nachteil bei der Pervaporation ist der durch den Phasenwechsel der permeierenden Komponenten (Verdampfung) bedingte Temperaturabfall in Strömungsrichtung des Feeds. Die Verdampfungsenthalpie des Permeats wird dem Feedgemisch entzogen, so daß es zu einer Temperaturabsenkung in Strömungsrichtung des Feeds kommt. Durch die Absenkung der Temperatur nimmt im allgemeinen der Permeatfluß stark ab, so daß zur Aufrechterhaltung der Membrantrennleistung das Feedgemisch zwischenzeitlich aufgewärmt werden muß (EP-A 294 827). Dieser Umstand führt insbesondere bei kontinuierlich geführten Prozessen zu einer aufwendigen Verschaltung von Membranmodulen und Wärmetauschern und damit zu hohen Investitionen.

Die Dampfpermeation wird unter anderem in EP-A-299 577 bei der Herstellung von Alkoholaten angewandt und ist in der Literatur "Industrial Application of Vapour Permeation" (U. Sander, H. Janssen, Journal of Membrane Science, 61 (1991), S. 113-129) beschrieben. Bei der Dampfpermeation wird der Feed im Gegensatz zur Pervaporation dampfförmig über die Membran geführt, d.h. über der Pervaporation auftretende Abkühlung des Feeds in Strömungsrichtung aufgrund des Phasenwechsels von Feed zu Permeat tritt nicht auf. Bei der Dampfpermeation kann somit auf die aufwendige Verschaltung von Membranmodulen und Wärmetauschern verzichtet werden.

Andererseits ist bei der Dampfpermeation nachteilig, daß der Permeatfluß im überhitzten Zustand des Dampfes (Feed) stark abnimmt. Eine Überhitzung des Dampfes läßt sich jedoch aufgrund der auftretenden Druckverluste in Strömungsrichtung in technischen Modulen nicht vermeiden. Es wird daher immer angestrebt, am sogenannten Sattdampfzustand des Feeds zu arbeiten, was beispielsweise dadurch erreicht werden kann, daß man den Feed zwischen den Modulen so weit verdichtet, daß bei Eintritt in den nachfolgenden Modul Sattdampfzustand erreicht wird. Nachteilig ist in diesem Fall jedoch, daß anstelle der bei Pervaporation erforderlichen Wärmetauscher Kompressoren zur Aufrechterhaltung der Membrantrennleistung nötig sind.

Eine reine Dampfpermeation wird in DE-A 4019170 beschrieben; zur Vermeidung der Vermischung von dampfförmiger und kondensierter Phase wird in den Feedzulauf ein Tropfenabscheider geschaltet. In EP-A 476370 wird die Erfindung in der Benutzung einer durch Plasmapolymerisation erhaltenen Membran gesehen; ansonsten wird das dortige Verfahren als Pervaporation oder Dampfpermeation durchgeführt, ohne eine Kombination beider Verfahren in Betracht zu ziehen.

Es wurde nun ein Verfahren zur Herstellung von Maleinsäuredialkylestern aus Maleinsäure, Maleinsäureanhydrid oder Maleinsäuremonoalkylestern mit C₁-C₈-Alkoholen, gegebenenfalls in Gegenwart eines sauren Katalysators, in der Siedehitze gefunden, das dadurch gekennzeichnet ist, daß man das entweichende Wasser/Alkohol/Dampfgemisch zusammen mit seiner kondensierten Phase als Feed an einer hydrophilen Membran entlangführt, an der Wasser ausgeschleust wird und den entwässerten Alkohol in die Reaktionsmischung zurückführt, wobei der Anteil der kondensierten Phase im Feed 5 bis 90 Gew.-% beträgt.

Als Alkoholkomponenten können in dem erfindungsgemäßen Verfahren geradkettige oder verzweigte, offenkettige oder cyclische, gesättigte oder ungesättigte C₁-C₈-Alkohole eingesetzt werden. Besonders bevorzugt sind solche Alkohole, die mit Wasser ein Azeotrop bilden und dadurch das destillative Trennverfahren für die Rückführung der Alkoholkomponente unmöglich machen, wie z.B. Ethanol, n-Propanol und iso-Propanol.

Die Reaktionstemperaturen richten sich naturgemäß auch nach der Beständigkeit der eingesetzten Membran. In der Regel wird bei Temperaturen von 50 bis 150°C, bevorzugt bei 70 bis 130°C, besonders bevorzugt bei der Siedetemperatur der Alkoholkomponente gearbeitet.

In einer der möglichen Ausführungsformen können diese Temperaturen jedoch durch Arbeiten unter Druck oder Vakuum erhöht bzw. erniedrigt werden. Erhöhte Temperaturen und damit einhergehende Druckerhöhungen können sich beispielsweise dann als vorteilhaft erweisen, wenn das Alkohol/Wasserdampfgemisch bei Normaldruck eine Temperatur besitzt, die unterhalb der optimalen Arbeitstemperatur der verwendeten Membran liegt.

Die verwendeten Membranen sind beispielsweise in EP-592 883 beschreiben und können beispielsweise aus Cellulosediacetat, Polyimid, Cellulosetriacetat oder Polyvinylalkohol hergestellt werden oder eine durch Plasmapolymerisation hergestellte porenfreie Schicht darstellen. Die Polymermaterialien haben hierbei im allgemeinen ein Molekulargewicht zwischen 15 000 und 200 000. Polyvinylalkohol wird im allgemeinen durch weitgehende Verseifung von Polyvinylacetat hergestellt; die Verseifungsgrade sollen bevorzugt über 95 %, besonders bevorzugt über 98 % liegen. Wegen der Wasserlöslichkeit von Polyvinylalkohol wird dies im allgemeinen in vernetzter Form eingesetzt. Eine solche Vernetzung kann in einer Veretherung, Veresterung oder Acetalisierung mit mehrfunktionellen Verbindungen bestehen.

In bevorzugter Form werden Compositmembranen eingesetzt, die im allgemeinen aus mehreren Schichten, nämlich einer Trägerschicht, einer porösen Schicht und der eigentlichen Trennschicht bestehen. Als Trägerschicht kommen im allgemeinen hochporöse flexible Gewebe oder Vliese aus Fasern, einschließlich Metallfasern, Polyolefinen, Polysulfonen, Polyetherimiden, Polyphenylsulfiden oder Kohlenstoff in Frage; gleichfalls geeignet sind poröse Strukturen aus Glas, Keramik, Graphit oder Metallen. Die poröse Stützschicht hat bevorzugt eine asymmetrische Porenstruktur. Solche porösen Stützschichten können beispielsweise aus Polysulfon, Polyethersulfon, Polyetherimid, Polyvinylidenfluorid, hydrolysiertem Cellulosetriacetat, Polyphenylensulfid, Polyacrylnitril, Polyester, Polytetrafluorethylen, Polyethylen, Polyvinylalkohol, Copolymeren von trifluorierten Polyolefinen sowie anderen geeigneten Polymerisaten hergestellt werden. Die Molekulargewichte können gleichfalls im Bereich von 15 000 bis 200 000 liegen. Die eigentliche Trennschicht kann wiederum aus Cellulosediacetat, Cellulosetriacetat, Polyvinylalkohol oder durch Plasmapolymerisation hergestellten Schicht bestehen. Polyvinylalkohol wird in der oben beschriebenen Weise vernetzt, um dem Angriff von Wasser bei höheren Temperaturen besser zu widerstehen. Die Membranen können als Wickelmodul, Plattenmodul, Kissenmodul, Hohlfaser- oder Kapillarmodul eingesetzt werden. Besonders bevorzugt werden Wickelmodule.

Das Verfahren wird so durchgeführt, daß der Feed dem Membranmodul als Gemisch aus Dampf und kondensierter Phase zugeführt wird. Dabei wird ein Teil des Dampfes, vorzugsweise in einem dem Modul vorgeschalteten Wärmetauscher kondensiert, so daß der Feed einen Anteil an kondensierter Phase von 5-90 Gew.-% besitzt, vorzugsweise 5-50 Gew.-%, insbesondere 10-20 Gew.-%.

Eine besonders vorteilhafte Entwässerung des Feeds kann erreicht werden, wenn der als Retentat aus dem Membranmodul austretende Feed-Dampf im Sattdampfzustand ist.

Die vorzugsweise kontinuierlich verlaufende Rückführung des entwässerten Alkohols (Retentat) in die Reaktionsmischung kann entweder direkt als Dampf (durchblasen) oder in Form der, gegebenenfalls durch zwischengeschaltete Destillationskolonnen erhaltenen, kondensierten Phase erfolgen. Bevorzugt ist eine dampfförmige Rückführung.

Als gegebenenfalls einzusetzender saurer Katalysator können sowohl anorganisch wie organische Säuren verwendet werden; beispielsweise Schwefelsäure oder p-Toluolsulfonsäure. Die möglichen Mengen des Katalysators bewegt sich dabei in der für Veresterungsreaktionen üblichen Bereichen.

Das Verfahren kann im übrigen diskontinuierlich oder kontinuierlich durchgeführt werden, wobei die kontinuierliche Fahrweise in einem Rührkessel oder bevorzugt einer Reaktionskolonne durchgeführt werden kann.

In einer weiteren bevorzugten Ausführungsform wird der Feed (als Dampf) unter Sattdampfbedingungen an der Membran entlang geführt.

Das erfindungsgemäße Verfahren wird in einer bevorzugten Ausführungsform im allgemeinen so durchgeführt, daß in einem Reaktionskessel geschmolzenes oder festes Maleinsäureanhydrid zu vorgelegtem Alkohol gegeben wird. Dabei bildet sich spontan der Maleinsäurehalbester. Nach Zugabe des sauren Katalysators wird aufgeheizt bis die Mischung siedet und das abdestillierende Alkohol/Wasser-Gemisch direkt oder über eine kurze Kolonne dem Membranmodul dampfförmig zugeführt. Dem Modul ist ein Wärmetauscher vorgeschaltet, um einen Teil des Dampfes zu kondensieren und die kondensierte Phase dem Feed beizumischen. Das Permeat wird abgezogen und das Retentat in die Reaktionsmischung kontinuierlich zurückgeführt. Nach beendeter Reaktion werden die sauren Bestandteile des Reaktionsansatzes (Katalysator, Monoester) mit einer Base neutralisiert und der überschüssige Alkohol abdestilliert. Dieser kann beim nächsten Ansatz als Vorlage wiederverwendet werden. Anschließend wird der Maleinsäurediester destilliert.

Nach diesem Verfahren wird der Diester in Ausbeuten von über 95 % und in hohen Reinheiten (größer 99 %), d.h. im wesentlichen frei von Isomerisierungs- und Additionsprodukten, erhalten.

Die bisher in der Literatur beschriebenen Verfahren zur Herstellung von Maleinsäuredialkylestern gehen, wie eine bevorzugte Ausführungsform des erfindungsgemäßen Verfahrens, von Maleinsäureanhydrid und den entsprechenden Alkoholen aus. Aus den kinetischen Daten wird diese Reaktion, die unter Variation verschiedenster Parameter wie Art und Menge des Katalysators, Molverhältnisse der Ausgangsstoffe, Reaktionszeit und -temperatur untersucht wurde, ergibt sich für die Reaktion mit Ethanol ein Gleichgewichtsverhältnis zwischen Mono-und Diethylester von 24 : 76. Um dieses Gleichgewicht nun zu verschieben, mußten bisher entweder größere Mengen Ethanol eingesetzt werden oder das Wasserazeotrop mit Hilfe eines Schleppmittels entfernt werden. Durch Einsatz des Membranmoduls im erfindungsgemäßen Verfahren gelingt es überraschenderweise ohne großen Aufwand, während der gleichen Reaktionszeit die Umwandlung in den Diethylester quantitativ zu bewerkstelligen.

Die Anwendung der Membrantechnik bei dem beschriebenen Verfahren stellt wegen der speziellen Problematik der Maleinsäureanhydridveresterung (Rückbildung von flüchtigem Maleinsäureanhydrid aus dem Halbester) die Frage nach der Beständigkeit der Membran. Es ist nämlich zu erwarten gewesen, daß mitgerissener Halbester oder Maleinsäureanhydrid die Membran schädigen und ihr nur kurze Standzeiten geben, was wirtschaftlich nicht mehr zu vertreten wäre. Der Einsatz der Pervaporation oder Dampfpermeation ist mit den oben beschriebenen Nachteilen, nämlich den aufwendigen Verschaltungen von Membranmodulen und Wärmetauschern bzw. Membranmodulen und Kompressoren verbunden. Insbesondere bei einer direkten Verschaltung von Destillation und Membrantrenneinheit ist mit erhöhtem Aufwand zu rechnen.

Das beschriebene Verfahren führt somit auf einfach durchzuführende Weise zu verbesserten Ausbeuten an Maleinsäuredialkylestern ohne größere, störende Mengen an Nebenprodukten wie Fumarsäure- oder Alkoxybernsteinsäureestern. Die Reaktionszeiten können beträchtlich verkürzt werden. Weiterhin kann auf größere Überschüsse an Alkohol oder die Verwendung von Schleppmitteln verzichtet werden, die nach Beendigung der Reaktion in einem zusätzlichen Verfahrensschritt recycliert werden müssen. Bevorzugt wird der Alkohol in einem molaren Verhältnis zu jeder zu veresternden Säurefunktion von 10:1 bis 1:1, vorzugsweise 1,1:1 bis 1,4:1, eingesetzt. Das molare Verhältnis von Maleinsäureanhydrid zu Alkohol beträgt vorzugsweise 1:2,2 bis 1:2,5. Die Abwassermengen, die z.B. bei der Abtrennung von sauren Bestandteilen (Maleinsäurehalbester) durch Neutralisation entstehen, werden durch das erfindungsgemäße Verfahren ebenfalls erheblich verringert.

Die nach dem erfindungsgemäßen Verfahren hergestellten Maleinsäuredialkylester dienen als Vorprodukte für Wirkstoffe, Netzmittel oder Lackhärter.

Das erfindungsgemäße Verfahren sei an folgenden Beispielen demonstriert.

### Beispiel

### Beispiel 1

In einem 1,6 m³ Stahl-Emaille-Rührwerkskessel mit Destillationskolonne, Wärmetauscher und aufgesetztem Dampfpermeationsmodul (54 m² Wickelmodul, Polyvinylalkohol von Typ Texsep® 1 auf Ultem® der Fa. Texaco) werden 467 kg Ethanol vorgelegt und 328 kg Maleinsäureanhydrid sowie 3,3 kg p-Toluolsulfonsäure zugegeben. Der Kesselinhalt wird auf 100°C Sumpftemperatur erhitzt und das abdestillierende Ethanol/Wasser-Gemisch über die Kolonne im Wärmetauscher und ins Dampfpermeationsmodul geleitet. Der Feed wird dabei so eingestellt, daß der Dampfanteil ca. 80 Gew.-% und der Anteil der kondensierten Phase etwa 20 Gew.-% beträgt. Das Permeat (147 kg in 11 Stunden, davon 61,7 kg Wasser und 85,3 kg Ethanol) wird abgezogen und das Retentat (größer 99 % Ethanol) dampfförmig in den Rührwerkskessel zurückgeführt. Nach 11 Stunden (GC-Probe) liegt der Umsatz bei über 95 % der Theorie. Nach Zugabe von 1 kg Natriumhydrogencarbonat wird das überschüssige Ethanol über die Kolonne abdestilliert und als Hauptfraktion der Maleinsäurediethylester gewonnen (Siedepunkt 120°C, 20 mbar). Die Ausbeute liegt bei 95 % (546kg, die Reinheit bei 99 %).

### Beispiel 2

In einem 10 l Glasbehälter mit Destillationskolonne, Wärmetauscher und aufgesetztem Dampfpermeationsmodul (0,5 m² Wickelmodul, Polyvinylalkohol, vom Typ Texsep® 4 der Texaco) werden 6,5 kg Isopropanol (IPA) vorgelegt und 3,5 kg Maleinsäureanhydrid sowie 0,04 kg Schwefelsäure zugegeben. Der Kesselinhalt wird auf 100°C Sumpftemperatur erhitzt und das abdestillierende IPA/Wasser-Gemisch über die Kolonne im Wärmetauscher und ins Dampfpermeationsmodul geleitet. Der Feed wird dabei so eingestellt, daß der Dampfanteil ca. 80 Gew.-% und der Anteil an kondensierter Phase etwa 20 Gew.-% beträgt. Das Permeat (0,7 kg in 5 Stunden, davon 0,67 kg Wasser und 0,3 kg IPA) wird abgezogen und das Retentat (größer 99 % IPA) in der Rührwerkskessel zurückgeführt. Nach 10 Stunden (GC-Probe) liegt der Umsatz bei über 95 % der Theorie. Nach Zugabe von 0,1 kg Natriumhydrogencarbonat wird der überschüssige IPA über die Kolonne abdestilliert und als Hauptfraktion der Maleinsäurediisopropylester gewonnen. Die Ausbeute liegt bei 95 % (6,8 kg, die Reinheit bei 99 %).

## Patentansprüche

1. Verfahren zur Herstellung von Maleinsäuredialkylestern aus Maleinsäure, Maleinsäureanhydrid oder Maleinsäuremonoalkylestern durch Umsetzung mit C₁-C₈-Alkoholen, in Gegenwart eines sauren Katalysators in der Siedehitze, dadurch gekennzeichnet, daß man das entweichende Wasser/Alkohol/Dampfgemisch zusammen mit seiner kondensierten Phase als Feed an einer hydrophilen Membran entlangführt, an der Wasser ausgeschleust wird und den entwässerten Alkohol in die Reaktionsmischung zurückführt, wobei der Anteil der kondensierten Phase im Feed 5 bis 90Gew.-% beträgt.

2. Verfahren nach Anspruch 1, dadurch gekennzeichnet, daß als C₁-C₈-Alkohole solche eingesetzt werden, die mit Wasser ein Azeotrop bilden.

3. Verfahren nach Anspruch 1, dadurch gekennzeichnet, daß als C₁-C₈-Alkohol Ethanol eingesetzt wird.

4. Verfahren nach Anspruch 1, dadurch gekennzeichnet, daß man von Maleinsäureanhydrid und Ethanol ausgeht.

5. Verfahren nach Anspruch 1, dadurch gekennzeichnet, daß die verwendete Membran aus Cellulosediacetat, Cellulosetriacetat, Polyvinylalkohol oder Polyamid hergestellt worden ist oder eine durch Plasmapolymerisation hergestellte porenfreie Schicht darstellt.

6. Verfahren nach Anspruch 1, dadurch gekennzeichnet, daß als Membran eine Composit-Membran eingesetzt wird.

7. Verfahren nach Anspruch 1, dadurch gekennzeichnet, daß die Membran in Form eines Wickelmoduls eingesetzt wird.

8. Verfahren nach Anspruch 1, dadurch gekennzeichnet, daß das molare Verhältnis von Alkohol zu jeder der veresternden Säurefunktionen 10:1 bis 1:1, insbesondere 1,1:1 bis 1,4:1 beträgt.

9. Verfahren nach Anspruch 1, dadurch gekennzeichnet, daß der Anteil der kondensierten Phase im Feed 5-50 Gew.-%, insbesondere 10-20 Gew.-% beträgt.

## Claims

1. Process for the preparation of dialkyl maleates from maleic acid, maleic anhydride or monoalkyl maleates by reaction with C₁-C₈-alcohols in the presence of an acidic catalyst and at boiling heat, characterized in that the escaping water/alcohol/vapour mixture, together with its condensed phase, is conducted as feed along a hydrophilic membrane at which it is purged of water and the dehydrated alcohol is returned to the reaction mixture, the portion of the condensed phase in the feed being 5 to 90 % by weight.

2. Process according to Claim 1, characterized in that the C₁-C₈-alcohols used are those which form an azeotrope with water.

3. Process according to Claim 1, characterized in that the C₁-C₈-alcohol used is ethanol.

4. Process according to Claim 1, characterized in that maleic anhydride and ethanol are used as starting materials.

5. Process according to Claim 1, characterized in that the membrane used has been produced from cellulose diacetate, cellulose triacetate, poly(vinyl alcohol) or polyamide or represents a pore-free layer produced by plasma polymerization.

6. Process according to Claim 1, characterized in that the membrane used is a composite membrane.

7. Process according to Claim 1, characterized in that the membrane is used in the form of a coil-wound module.

8. Process according to Claim 1, characterized in that the molar ratio of alcohol to each of the esterifying acid functions is 10:1 to 1:1, in particular 1.1:1 to 1.4:1.

9. Process according to Claim 1, characterized in that the portion of the condensed phase in the feed is 5-50 % by weight, in particular 10-20 % by weight.

## Revendications

1. Procédé pour la préparation de maléates de dialkyle à partir de l'acide maléique, de l'anhydride maléique ou de maléates de monoalkyle par réaction avec des alcools en C₁-C₈ en présence d'un catalyseur acide et à la chaleur du bouillon, caractérisé en ce que l'on envoie le mélange de vapeurs eau/alcool qui se dégage, avec sa phase condensée, en tant que courant d'alimentation devant une membrane hydrophile sur laquelle on sépare l'eau et d'où l'on recycle l'alcool déshydraté dans le mélange de réaction, la proportion de la phase condensée dans le courant d'alimentation représentant de 5 à 90 % en poids.

2. Procédé selon la revendication 1, caractérisé en ce que l'on utilise des alcools en C₁-C₈ qui forment un azéotrope avec l'eau.

3. Procédé selon la revendication 1, caractérisé en ce que l'alcool en C₁-C₈ est l'éthanol.

4. Procédé selon la revendication 1, caractérisé en ce que l'on part de l'anhydride maléique et de l'éthanol.

5. Procédé selon la revendication 1, caractérisé en ce que la membrane utilisée a été préparée en diacétate de cellulose, en triacétate de cellulose, en alcool polyvinylique ou en polyamide ou consiste en une couche non poreuse préparée par polymérisation en plasma.

6. Procédé selon la revendication 1, caractérisé en ce que l'on utilise une membrane composite.

7. Procédé selon la revendication 1, caractérisé en ce que la membrane est à l'état de module en rouleau.

8. Procédé selon la revendication 1, caractérisé en ce que le rapport molaire de l'alcool avec chacune des fonctions acides à estérifier va de 10:1 à 1:1 et plus spécialement de 1,1:1 à 1,4:1.

9. Procédé selon la revendication 1, caractérisé en ce que la proportion de la phase condensée dans le courant d'alimentation est de 5 à 50 % en poids, plus spécialement de 10 à 20 % en poids.
